# EUROPÄISCHE PATENTANMELDUNG

(11) **EP 4 360 573 A1**
(43) Veröffentlichungstag der Anmeldung: **01.05.2024**
(21) Anmeldenummer: 22204530.4
(22) Anmeldetag: 28.10.2022
(51) Int. Cl.: A61B 18/12, A61B 18/14, A61B 18/16

(54) **ELEKTROCHIRURGISCHES SYSTEM**

(71) Anmelder: Erbe Elektromedizin GmbH, 72072 Tübingen (DE)
(72) Erfinder: MAIER, Philipp, 72074 Tuebingen (DE)
(74) Vertreter: Rüger Abel Patentanwälte PartGmbB

(57) **Zusammenfassung**

Die Erfindung betrifft ein Versorgungsgerät (11) für ein elektrochirurgisches System (10) sowie ein elektrochirurgisches System (10), aufweisend ein erfindungsgemäßes Versorgungsgerät (11). Das Versorgungsgerät (11) hat zusätzlich zu einem Generator (31) eine Messsignalquelle (34), die ein Messsignal (XM) in Form einer Wechselspannung und/oder eines Wechselstromes bereitstellen kann. Ein sich aufgrund des Messsignals (34) ergebender Impedanzparameter (P) kann ausgewertet werden. Eine Schalteinrichtung (51) des Versorgungsgeräts (11) ist zwischen mehreren Schaltzuständen (C1-C5) umschaltbar. In den unterschiedlichen Schaltzuständen (C1-C5) lassen sich unterschiedliche Auswertefunktionen mittels der Auswerteeinheit (50) realisieren. Die Messsignalquelle (34) kann sowohl ein Messsignal an einen Behandlungsstrompfad (B) an einem Arbeitsanschluss (21) des Versorgungsgeräts (11) als auch an einen Neutralstrompfad (N) zwischen Neutralanschlüssen (26, 27) des Versorgungsgeräts (11) anlegen. Dadurch kann mittels des Messsignals (XM) sowohl ein behandeltes Gewebe (16) analysiert als auch ein Kontakt zwischen der Neutralelektrode (13) und dem Patienten geprüft bzw. überwacht werden.

## Beschreibung

Die Erfindung betrifft ein elektrochirurgisches System sowie ein Verfahren zu dessen Betrieb.

Es ist bei elektrochirurgischen Systemen bekannt, Gewebe mittels Impedanzspektroskopie zu analysieren, beispielsweise, um gesundes Gewebe von Tumorgewebe zu unterscheiden. Hierzu wird mittels eines Messstromes zwischen zwei Elektroden die Impedanz des Gewebes ermittelt und ausgewertet.

Ein derartiges Verfahren ist beispielsweise aus EP 0 813 387 A1 bekannt. Zwischen mehreren Elektroden eines Instruments eines elektrochirurgischen Systems kann entweder eine Messspannung oder eine gegenüber der Messspannung höhere Behandlungsspannung angelegt werden. Mittels der Messspannung können Impedanzmessungen zur Gewebebestimmung durchgeführt werden. Mittels der Behandlungsspannung kann Gewebe behandelt werden. In einem Versorgungsgerät, zur Versorgung des Instruments mit der Messspannung oder der Behandlungsspannung, kann eine Umschalteinrichtung vorhanden sein, um zwischen einer Energiequelle für die Messspannung und einer Energiequelle für die Behandlungsspannung umschalten zu können.

EP 1 511 534 B1 beschreibt eine Vorrichtung zum elektrochirurgischen Veröden von Körpergewebe. Die Vorrichtung hat ein Versorgungsgerät, an das mehrere Elektroden angeschlossen sind. Mittels eines Hochfrequenzgenerators des Versorgungsgeräts wird ein Stromfluss zwischen jeweils zwei Elektroden erzeugt, um das Gewebe zu koagulieren. Mithilfe einer Messeinrichtung kann eine Impedanz zwischen zwei Elektroden ermittelt und aus der Anzahl der vorhandenen Elektroden können mindestens zwei aktive Elektroden basierend auf der Impedanzmessung ausgewählt werden.

Das elektrochirurgische System gemäß WO 2012/151 493 A2 weist ein Versorgungsgerät und ein daran angeschlossenes Instrument auf. Mittels eines Schalters kann wahlweise eine Auswerteeinrichtung zur Impedanzanalyse des Gewebes oder ein Generator zur Erzeugung einer hochfrequenten Behandlungsspannung mit dem Instrument verbunden werden. Über die Auswerteeinrichtung werden Frequenzen identifiziert, die die Impedanz verringern und damit die Leitfähigkeit des Gewebes erhöhen. Anschließend wird die Behandlungsspannung bei den identifizierten Frequenzen an das Instrument angelegt.

Eine Impedanzmesseinrichtung bei einem elektrochirurgischen System ist außerdem in DE 10 2016 220 157 A1 beschrieben. Basierend auf Impedanzmessungen bei unterschiedlichen Frequenzen, kann ein Zustand des behandelten Gewebes ermittelt werden. Beispielsweise soll eine ungewollte Karbonisierung von Gewebe oder das Anhaften von Gewebe an den Elektroden des elektrochirurgischen Instruments vermieden werden.

DE 10 2018 114 482 A1 sowie DE 197 14 972 A1 beschreiben elektrochirurgische Systeme mit einem monopolaren Instrument und einer Neutralelektrode, die an ein Versorgungsgerät angeschlossen sind. Über eine Impedanzmessung wird bei DE 10 2018 114 482 A1 ein Neutralelektrodentyp ermittelt, während DE 197 14 972 A1 vorschlägt, den Kontakt der Neutralelektrode am Körper des Patienten anhand einer gemessenen Gewebeimpedanz zu überwachen. Bei beiden Anwendungen erfolgt die Messung der Gewebeimpedanz bei mehreren Frequenzen.

EP 3 496 638 A1 offenbart ein elektrochirurgisches System mit einer Messeinheit. Das elektrochirurgische System hat ein Versorgungsgerät und wenigstens zwei Elektroden, die an das Versorgungsgerät angeschlossen sind und zwischen denen eine Wechselspannung zur Behandlung von Gewebe angelegt werden kann. Zusätzlich ist es möglich, eine Messspannung zwischen den Elektroden bei einer von der Behandlungs-Wechselspannung verschiedenen Frequenz anzulegen und daraus eine Gewebeeigenschaft des Gewebes zwischen den Elektroden zu ermitteln.

Ausgehend vom Stand der Technik kann es als Aufgabe der vorliegenden Erfindung angesehen werden, ein verbessertes Versorgungsgerät sowie ein verbessertes elektrochirurgisches System zu schaffen, das bei einfachem und kostengünstigem Aufbau eine Gewebeanalyse sowie einen Kontakt einer Neutralelektrode am Patienten ermöglicht.

Diese Aufgabe wird durch ein Versorgungsgerät mit den Merkmalen des Patentanspruches 1 sowie ein elektrochirurgisches System mit den Merkmalen des Patentanspruches 15 gelöst.

Das Versorgungsgerät gemäß der vorliegenden Erfindung ist für den Einsatz in einem elektrochirurgischen System eingerichtet und vorgesehen. Es weist mehrere Geräteanschlüsse zum Anschluss eines elektrochirurgischen Instruments und zum optionalen Anschluss einer Neutralelektrode auf. Zu den Geräteanschlüssen gehören zwei Neutralanschlüsse zum Anschließen einer Neutralelektrode und wenigstens ein Arbeitsanschluss für jeweils eine Arbeitselektrode eines elektrochirurgischen Instruments. Weist das Versorgungsgerät mindestens zwei Arbeitsanschlüsse auf, können an die Arbeitsanschlüsse bipolare (oder auch andere multipolare) Instrumente angeschlossen werden. Beim Anschließen von einem monopolaren Instrument genügt ein Arbeitsanschluss für die Arbeitselektrode des monopolaren Instruments. In diesem Fall werden die Neutralanschlüsse zum Anschließen der Neutralelektrode verwendet, die beim Einsatz eines bipolaren Instruments nicht benötigt wird.

Das Versorgungsgerät hat außerdem einen Generator zur Bereitstellung einer Generatorspannung und/oder eines Generatorstromes. Bei der Generatorspannung handelt es sich um eine Hochfrequenzspannung und/oder bei dem Generatorstrom handelt es sich um einen hochfrequenten Strom. Die Frequenz der Generatorspannung und/oder des Generatorstromes beträgt insbesondere mindestens 200kHz. Der Generatorstrom bzw. die Generatorspannung werden der wenigstens einen Arbeitselektrode eines elektrochirurgischen Instruments bereitgestellt, um biologisches Gewebe zu behandeln, beispielsweise zu koagulieren oder zu schneiden.

Das Versorgungsgerät verfügt außerdem über eine Messsignalquelle zur Bereitstellung eines Messsignals. Bei dem Messsignal handelt es sich um ein Wechselspannungssignal oder ein Wechselstromsignal mit vorzugsweise alternierender Polarität. Dementsprechend kann die Messsignalquelle eine Spannungsquelle oder eine Stromquelle sein. Das Messsignal hat eine Messfrequenz, die vorzugsweise in einem Bereich bis maximal 100 MHz liegt. Optional kann die Messfrequenz mindestens 10 Hz, mindestens 50 Hz oder mindestens 100 Hz betragen. Die Messfrequenz ist insbesondere veränderbar. Vorzugsweise können zwei oder mehr unterschiedliche Messfrequenzen für das Messsignal ausgewählt oder eingestellt werden.

Das Versorgungsgerät hat außerdem eine Messeinrichtung zur Messung eines Impedanzparameters, der eine Impedanz zwischen jeweils zwei der vorhandenen Geräteanschlüssen beschreibt, denen das Messsignal bereitgestellt wird. Zwischen diesen ausgewählten Geräteanschlüssen liegt eine Messspannung an bzw. es fließt ein Messstrom. Abhängig davon, ob ein monopolares oder bipolares Instrument verwendet wird, sind die beiden Geräteanschlüsse über eine erste Arbeitselektrode, das Gewebe eines Patienten und eine zweite Arbeitselektrode elektrisch leitend miteinander verbunden (bipolares Instrument) oder über wenigstens eine Arbeitselektrode, das Gewebe eines Patienten und eine Neutralelektrode elektrisch leitend miteinander verbunden (monopolares Instrument). In beiden Fällen hängt der Impedanzparameter unter anderem von der Impedanz des Gewebes des Patienten ab.

Zur Messung wenigstens eines Stromes und/oder wenigstens einer Spannung kann die Messeinrichtung einen oder mehrere Stromsensoren und/oder Spannungssensoren aufweisen.

Das Versorgungsgerät hat außerdem eine Schalteinrichtung. Die Schalteinrichtung ist zwischen mehreren Schaltzuständen und beispielsgemäß zumindest zwischen einem ersten Schaltzustand und einem zweiten Schaltzustand umschaltbar.

Im ersten Schaltzustand verbindet die Schalteinrichtung die Messsignalquelle einerseits mit dem ersten der zwei Neutralanschlüsse und andererseits mit dem zweiten der zwei Neutralanschlüsse. Somit kann eine Messspannung zwischen den zwei Neutralanschlüssen angelegt werden oder es kann bei angeschlossener Neutralelektrode ein Messstrom vom ersten Neutralanschluss zum zweiten Neutralanschluss bzw. umgekehrt durch die Neutralelektrode fließen.

Im ersten Schaltzustand ist die Messsignalquelle derart vom zumindest einen Arbeitsanschluss getrennt, dass eine an den zumindest einen Arbeitsanschluss angeschlossene Arbeitselektrode kein Messsignal erhält.

Im zweiten Schaltzustand hingegen ist die Messsignalquelle elektrisch mit dem zumindest einen Arbeitsanschluss verbunden. Das Messsignal wird der zumindest einen Arbeitselektrode eines angeschlossenen elektrochirurgischen Instruments bereitgestellt. Im Falle eines bipolaren Instruments kann basierend auf dem Messsignal ein Strom von der einen Arbeitselektrode über das Gewebe zur anderen Arbeitselektrode und von dort zurück zur Messsignalquelle fließen, während bei einem angeschlossenen monopolaren Instrument sowie einer angeschlossenen Neutralelektrode basierend auf dem Messsignal ein Strom von der wenigstens ei*nen* Arbeitselektrode über das Gewebe des Patienten zur Neutralelektrode und von dort zurück zur Messsignalquelle fließen kann. Unabhängig vom Typ des verwendeten Instruments wird über wenigstens eine Arbeitselektrode und das Gewebe des Patienten im zweiten Schaltzustand ein geschlossener Stromkreis bereitgestellt, der die Messsignalquelle aufweist.

Das Versorgungsgerät hat außerdem eine Auswerteeinheit, die mit der Messeinrichtung kommunikationsverbunden ist. Die Auswerteeinheit ist dazu eingerichtet, den gemessenen Impedanzparameter auszuwerten. Abhängig vom Auswerteergebnis besteht daher die Möglichkeit, das Versorgungsgerät zu steuern und/oder mittels einer Bedienschnittstelle des Versorgungsgerätes Informationen an den Anwender, beispielsweise einen Chirurgen, auszugeben.

Über die Schalteinrichtung kann somit das Messsignal der Messsignalquelle entweder einen Stromfluss durch die Neutralelektrode (erster Schaltzustand) oder einen Stromfluss über eine Arbeitselektrode, ein behandeltes Gewebe und über eine weitere Arbeitselektrode oder alternativ eine angeschlossene Neutralelektrode zurück zur Messsignalquelle bewirken (zweiter Schaltzustand). Die Messsignalquelle ist somit sowohl einsetzbar, um einen Kontakt zwischen der Neutralelektrode und einem Patienten zu überwachen bzw. zu prüfen (erster Schaltzustand), als auch um den Zustand und/oder den Typ eines zu behandelnden Gewebes eines Patienten zu ermitteln (zweiter Schaltzustand).

Mittels der Messsignalquelle kann demnach unabhängig von der Generatorspannung und/oder dem Generatorstrom eine Impedanzspektroskopie zur Ermittlung des Zustands und/oder des Typs eines behandelten Gewebes ausgeführt werden, und es kann der korrekten Kontakt bzw. die korrekten Anlage der Neutralelektrode am Patienten geprüft und/oder überwacht werden. Während der Anwendung des Versorgungsgeräts kann beispielsweise wiederholt zwischen dem ersten Schaltzustand und dem zweiten Schaltzustand umgeschaltet werden, so dass wiederholt das behandelte Gewebe analysiert, als auch die korrekte Anlage der Neutralelektrode geprüft und überwacht werden kann. Separate Spannungs- oder Stromquellen für die Impedanzspektroskopie einerseits und das Überwachen der Anlage einer Neutralelektrode andererseits können entfallen.

Bei einer bevorzugten Ausführung ist das Versorgungsgerät sowohl für den Anschluss eines monopolaren Instruments, als auch für den Anschluss eines bipolaren Instruments geeignet und eingerichtet.

Das Versorgungsgerät hat einen ersten Arbeitsanschluss zum Anschließen einer Arbeitselektrode eines monopolaren Instruments. Optional kann das Versorgungsgerät zusätzlich zum ersten Arbeitsanschluss einen zweiten Arbeitsanschluss zum Anschließen einer zweiten Arbeitselektrode aufweisen. Ein bipolares Instrument mit einer ersten Arbeitselektrode und einer zweiten Arbeitselektrode kann an den ersten Arbeitsanschluss und den zweiten Arbeitsanschluss angeschlossen werden. Das Versorgungsgerät eignet sich dann sowohl zum Betreiben von monopolaren, als auch zum Betreiben von bipolaren Instrumenten.

Bei einem bevorzugten Ausführungsbeispiel ist die Messsignalquelle als Wechselstromquelle ausgeführt und stellt eine eingeprägte Wechselspannung in Form einer Messspannung als Messsignal bereit. Alternativ kann als Messsignalquelle auch eine Wechselstromquelle verwendet werden. In beiden Fällen können die Wechselspannung bzw. der Wechselstrom bei unterschiedlichen Messfrequenzen bereitgestellt werden.

Im ersten Schaltzustand ist die Auswerteeinrichtung insbesondere dazu eingerichtet, basierend auf dem gemessenen Impedanzparameter einen elektrischen Kontakt zwischen einem Patienten und einer an die zwei neutralen Anschlüsse angeschlossenen Neutralelektrode zu prüfen bzw. zu überwachen. Dazu wird basierend auf dem Impedanzparameter insbesondere die Impedanz zwischen den beiden Neutralanschlüssen ermittelt. Die Impedanz kann bei einer einzigen oder bei mehreren verschiedenen Messfrequenzen des Messsignals ermittelt werden.

Bevorzugt hat die an die zwei Neutralanschlüsse angeschlossene Neutralelektrode wenigstens zwei elektrisch leitfähige Elektrodenbereiche, die innerhalb der Neutralelektrode nicht unmittelbar elektrisch miteinander verbunden sind. Daher können die Elektrodenbereiche der Neutralelektrode unterschiedliche elektrische Potentiale annehmen. Sie sind innerhalb der Neutralelektrode nicht elektrisch kurzgeschlossen oder niederohmig verbunden. Eine elektrische Verbindung zwischen den Elektrodenbereichen ist beim bestimmungsgemäßen Gebrauch der Neutralelektrode über den Patienten erreicht. Die Neutralelektrode wird insbesondere an einer geeigneten Stelle auf der Haut des Patienten angebracht, insbesondere aufgeklebt.

Durch die Bestimmung der Impedanz zwischen den beiden Neutralanschlüssen kann eine korrekte Anordnung mit ausreichend hoher Leitfähigkeit zwischen der Neutralelektrode und dem Patienten geprüft und überwacht werden. Ein unzureichender Kontakt kann einem Anwender des Versorgungsgeräts über eine geeignete Bedienschnittstelle angezeigt werden. Zusätzlich oder alternativ kann der Betrieb des Versorgungsgeräts unterbrochen werden, wenn festgestellt wird, dass der elektrisch leitende Kontakt zwischen der Neutralelektrode und einem Patienten während dem Einsatz des Versorgungsgeräts ungenügend ist. Bei beiden Varianten lassen sich Gewebeschäden durch zu große Stromdichten im Bereich der Neutralelektrode vermeiden.

Es ist außerdem vorteilhaft, wenn die Auswerteeinheit dazu eingerichtet ist, im zweiten Schaltzustand basierend auf dem gemessenen Impedanzparameter einen Gewebetyp zu bestimmen. Im zweiten Schaltzustand kann insbesondere eine Auswertung der Impedanz durchgeführt werden, die unter anderem vom zu behandelnden Gewebe abhängt, also insbesondere vom Zustand und/oder dem Typ des Gewebes. Die Impedanzermittlung im zweiten Schaltzustand kann bei einer oder mehreren Messfrequenzen des Messsignals erfolgen. Damit ist es möglich, einen Gewebetyp und/oder einen Zustand des zu behandelnden Gewebes zu ermitteln, mit dem die wenigstens eine Arbeitselektrode des Instruments in elektrisch leitendem Kontakt ist.

Es ist bevorzugt, wenn die Schalteinrichtung im ersten Schaltzustand und im zweiten Schaltzustand den Generator elektrisch derart von der Messsignalquelle und/oder den Geräteanschlüssen trennt, dass kein Generatorstrom zur Messsignalquelle und/oder den Geräteanschlüssen fließt.

Bei einer bevorzugten Ausführungsform ist der Generator dazu eingerichtet oder derart gesteuert, dass der Generator im ersten Schaltzustand und im zweiten Schaltzustand keine Generatorspannung und/oder keinen Generatorstrom erzeugt. Es wird keine Generatorspannung an den wenigstens einen Arbeitsanschluss angelegt und/oder es wird kein Stromfluss eines Generatorstromes über den wenigstens einen Arbeitsanschluss zu einer daran angeschlossenen Arbeitselektrode ermöglicht. Dadurch wird verhindert, dass eine Generatorspannung und/oder ein Generatorstrom die Auswertung des Impedanzparameters im ersten Schaltzustand und im zweiten Schaltzustand stört.

Bevorzugt verbindet die Schalteinrichtung im zweiten Schaltzustand die Messsignalquelle einerseits elektrisch mit dem zumindest einen Arbeitsanschluss und andererseits elektrisch mit den zwei Neutralanschlüssen. Die zwei Neutralanschlüsse werden im zweiten Schaltzustand innerhalb des Versorgungsgeräts durch die Schalteinrichtung niederohmig miteinander verbunden bzw. kurzgeschlossen. Die zwei Neutralanschlüsse haben daher im Wesentlichen dasselbe elektrische Potential.

Bei einer bevorzugten Ausführungsform kann die Schalteinrichtung in einen optionalen dritten Schaltzustand umgeschaltet werden, in dem die Schalteinrichtung den Generator einerseits elektrisch mit dem wenigstens einen Arbeitsanschluss verbindet und andererseits den Generator elektrisch mit den Neutralanschlüssen verbindet. In diesem dritten Schaltzustand wird bei angeschlossenem monopolarem Instrument und angeschlossener Neutralelektrode ein Stromfluss aufgrund der Generatorspannung und/oder des Generatorstromes zur Arbeitselektrode des Instruments, von dort durch in elektrisch leitenden Kontakt mit der Arbeitselektrode stehendem Gewebe und über die Neutralelektrode wieder zurück zum Versorgungsgerät ermöglicht. Dieser dritte Schaltzustand dient insbesondere der Behandlung des Gewebes mittels eines monopolaren Instruments, beispielsweise der Koagulation oder dem Schneiden des Gewebes.

Insbesondere ist die Auswerteeinheit dazu eingerichtet, im dritten Schaltzustand einen durch die Generatorspannung und/oder den Generatorstrom verursachten Stromfluss durch die zwei Neutralanschlüsse zu prüfen, beispielsweise, um einen ersten Strom vom ersten Neutralanschluss und einen zweiten Strom vom zweiten Neutralanschluss miteinander zu vergleichen. Bei diesem Vergleich können ein oder mehrere Stromparameter des ersten Stromes und des zweiten Stromes miteinander verglichen werden, beispielsweise eine Amplitude und/oder ein Betrag und/oder eine Phasenlage.

Die Messsignalquelle kann im dritten Schaltzustand zumindest phasenweise ein Messsignal bereitstellen. Insbesondere ist die Auswerteeinheit dazu eingerichtet, dass sie während eines monopolaren Behandlungsstromflusses auch den an den Neutralanschlüssen erfassten Impedanzparameter auswerten, um die die Übergangsimpedanz bzw. den elektrischen Kontakt zwischen der Neutralelektrode und dem Patienten zu prüfen bzw. zu überwachen. Die Messfrequenz ist dabei ausreichend verschieden von der Generatorfrequenz der Generatorspannung bzw. des Generatorstroms. Es kann ein mangelnder elektrischer Kontakt (zu große Übergangsimpedanz) auch während einer Aktivierung des Generators zwischen der Neutralelektrode und dem Patienten erkannt werden, beispielsweise verursacht durch eine zumindest teilweise Ablösung der Neutralelektrode vom Patienten. zu erkennen.

Bei einer bevorzugten Ausführungsform hat die Messeinrichtung eine frequenzabhängige Impedanzschaltung, die einen Parallelschwingkreis aufweisen oder ein Parallelschwingkreis sein kann. Der Parallelschwingkreis kann beispielsweise aus einer Parallelinduktivität und zwei Serienkondensatoren aufgebaut sein. Die frequenzabhängige Impedanzschaltung (beispielsweise der Parallelschwingkreis) verbindet den ersten Neutralanschluss mit dem zweiten Neutralanschluss verbindet.

Bevorzugt weist die Schalteinrichtung einen oder mehrere steuerbare Schalter auf, die die frequenzabhängige Impedanzschaltung vom ersten Neutralanschluss und/oder vom zweiten Neutralanschluss trennen, wenn sich die Schalteinrichtung im ersten Schaltzustand und/oder im zweiten Schaltzustand befindet. Vorzugsweise wird zumindest oder ausschließlich im dritten Schaltzustand eine elektrische Verbindung zwischen der frequenzabhängigen Impedanzschaltung und dem ersten Neutralanschluss und/oder dem zweiten Neutralanschluss hergestellt.

Es ist vorteilhaft, wenn im dritten Schaltzustand die Messsignalquelle dazu eingerichtet ist oder derart gesteuert wird, dass sich die Messfrequenz des Messsignals in einem vorgegebenen Frequenzbereich ändert. Dadurch ist es insbesondere möglich, eine Frequenz zu ermitteln, bei der die frequenzabhängige Impedanzschaltung ein lokales oder globales Impedanzmaximum aufweist.

Vorzugsweise kann die Schalteinrichtung in einem optionalen vierten Schaltzustand die Messsignalquelle elektrisch einerseits mit dem ersten Arbeitsanschluss und andererseits mit dem zweiten Arbeitsanschluss verbinden. Der vierte Schaltzustand ist für die Verwendung eines bipolaren Instruments vorgesehen und wird sozusagen analog zum zweiten Schaltzustand bei der Verwendung eines monopolaren Instruments verwendet. Die Auswerteeinheit ist dazu eingerichtet, im vierten Schaltzustand basierend auf dem gemessenen Impedanzparameter den Zustand und/oder den Typ des Gewebes zu bestimmen, mit dem die Arbeitselektroden des angeschlossenen Instruments in elektrisch leitendem Kontakt sind.

Vorzugsweise kann die Schalteinrichtung in einem optionalen fünften Schaltzustand den Generator mit dem ersten und dem zweiten Arbeitsanschluss verbinden und die Messsignalquelle von den Arbeitsanschlüssen trennen. Der fünfte Schaltzustand ist für die Verwendung eines bipolaren Instruments mit Generatorstrom vorgesehen.

Insbesondere ist die Messsignalquelle dazu eingerichtet oder wird derart gesteuert, dass sie im fünften Schaltzustand kein Messsignal erzeugt.

Zur Ansteuerung der Schalteinrichtung und optional der Messsignalquelle und/oder des Generators kann das Versorgungsgerät eine Steuereinheit aufweisen. Die Steuereinheit und die Auswerteeinheit können in einem einzigen Bauteil (insbesondere IC) integriert realisiert sein.

Die Kennzeichnung von Merkmalen, insbesondere von Bauteilen und Schaltzuständen, mit einem Zahlwort "erste(r)", "zweite(r)", usw. dient lediglich der Unterscheidung der Merkmale und impliziert keine Reihenfolge oder Priorisierung. Beispielsweise kann ein fünfter Schaltzustand vorhanden sein, auch wenn es keinen vierten Schaltzustand gibt.

Die Erfindung betrifft außerdem ein elektrochirurgisches System, aufweisend ein Versorgungsgerät nach irgendeinem der vorstehend erläuterten Ausführungsbeispiele. Zu dem elektrochirurgischen System gehört außerdem ein elektrochirurgisches Instrument sowie optional eine Neutralelektrode, wenn es sich bei dem elektrochirurgischen Instrument um ein monopolares Instrument handelt. Gehört zu dem elektrochirurgischen System ein bipolares Instrument, ist das bipolare Instrument an einen ersten Arbeitsanschluss und einen zweiten Arbeitsanschluss des Versorgungsgerätes angeschlossen. Gehört zu dem elektrochirurgischen System ein monopolares Instrument sowie eine Neutralelektrode, ist das monopolare Instrument an den Arbeitsanschluss oder an einen der vorhandenen Arbeitsanschlüsse angeschlossen und die Neutralelektrode ist an den ersten Neutralanschluss und den zweiten Neutralanschluss angeschlossen.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung basierend auf der beigefügten Zeichnung im Einzelnen erläutert. In der Zeichnung zeigen:
Figur 1 eine Prinzipdarstellung eines Ausführungsbeispiels eines elektrochirurgischen Systems aufweisend ein Versorgungsgerät, ein monopolares Instrument sowie eine Neutralelektrode,
Figur 2 eine Prinzipdarstellung eines weiteren Ausführungsbeispiels eines elektrochirurgischen Instruments aufweisend ein Versorgungsgerät sowie ein bipolares Instrument,
Figur 3 ein Ausführungsbeispiel einer Betriebsschaltung des Versorgungsgeräts aus Figuren 1 und 2 als Blockschaltbild, wobei die Betriebsschaltung einen Generator, eine Messsignalquelle, eine Messeinrichtung, eine Auswerteeinheit sowie eine Schalteinrichtung aufweist,
Figur 4 ein Ersatzschaltbild der Betriebsschaltung aus Figur 3 in einem ersten Schaltzustand der Schalteinrichtung,
Figur 5 ein Ersatzschaltbild der Betriebsschaltung aus Figur 3 in einem zweiten Schaltzustand der Schalteinrichtung,
Figur 6 ein Ersatzschaltbild der Betriebsschaltung aus Figur 3 in einem dritten Schaltzustand der Schalteinrichtung,
Figur 7 ein Ersatzschaltbild der Betriebsschaltung aus Figur 3 in einem vierten Schaltzustand der Schalteinrichtung,
Figur 8 ein Ersatzschaltbild der Betriebsschaltung aus Figur 3 in einem fünften Schaltzustand der Schalteinrichtung,
Figur 9 eine schematische, beispielhafte Darstellung eines zeitlichen Verlaufs eines Generatorstromes und eines Messstromes der Betriebsschaltung des Versorgungsgerätes im ersten und/oder zweiten und/oder vierten und/oder fünften Schaltzustand,
Figur 10 eine schematische, beispielhafte Darstellung eines zeitlichen Verlaufs eines Generatorstromes und eines Messstromes der Betriebsschaltung des Versorgungsgerätes im dritten Schaltzustand, und
Figur 11 ein beispielhafter Absolutwertverlauf einer Impedanz einer frequenzabhängigen Impedanzschaltung in Abhängigkeit von der Frequenz.

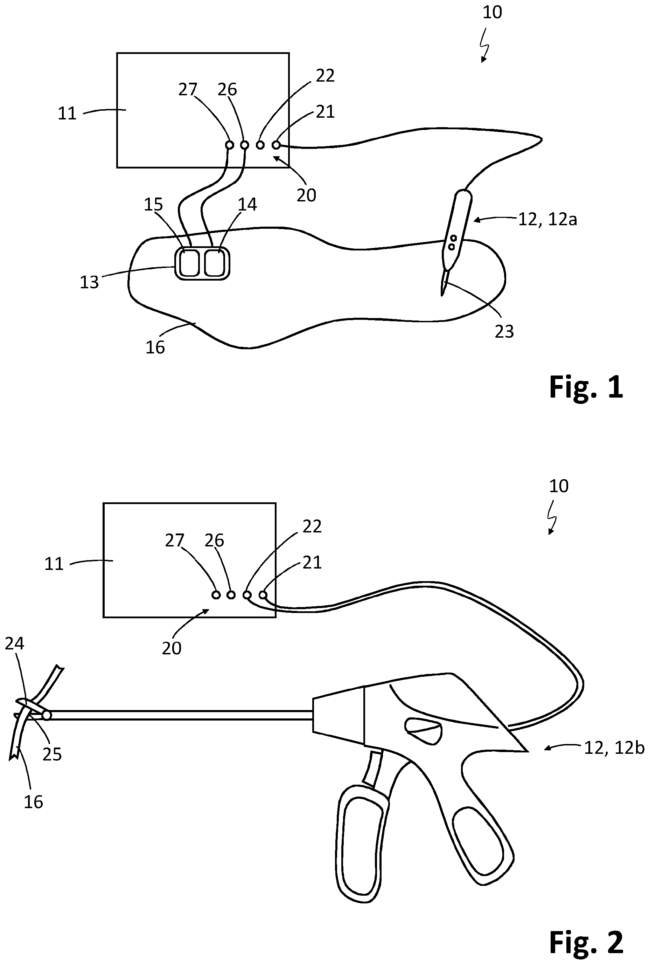

In den Figuren 1 und 2 sind beispielhaft Ausführungsbeispiele eines elektrochirurgischen Systems 10 dargestellt. Das elektrochirurgische System 10 hat ein Versorgungsgerät 11 entsprechend einer erfindungsgemäßen Ausführung. An das Versorgungsgerät 11 ist ein elektrochirurgisches Instrument 12 angeschlossen. Die in den Figuren 1 und 2 veranschaulichten elektrochirurgischen Instrumente sind für den offenchirurgischen Einsatz eingerichtet. Alternativ hierzu könnte das elektrochirurgische Instrument 12 auch für den endoskopischen oder minimalinvasiven Einsatz eingerichtet sein.

Das elektrochirurgische Instrument 12 kann ein monopolares Instrument 12a (Figur 1) oder ein bipolares Instrument 12b (Figur 2) sein. Bei der Verwendung eines monopolaren Instruments 12a weist das elektrochirurgische System außerdem eine Neutralelektrode 13 auf.

Bei dem hier veranschaulichten Ausführungsbeispiel hat die Neutralelektrode 13 einen elektrisch leitfähigen ersten Elektrodenbereich 14 sowie einen elektrisch leitfähigen zweiten Elektrodenbereich 15. Die beiden Elektrodenbereiche 14, 15 sind innerhalb der Neutralelektrode 13 nicht unmittelbar elektrisch niederohmig miteinander verbunden, so dass sie unterschiedliche elektrische Potentiale aufweisen können. Während des Einsatzes wird die Neutralelektrode 13 an einem zu behandelnden Patienten angebracht, so dass die Elektrodenbereiche 14, 15 elektrisch leitfähig mit dem Patienten verbunden sind. Beispielsweise kann die Neutralelektrode 13 auf die Haut des Patienten aufgeklebt werden. Eine elektrische Verbindung zwischen den beiden Elektrodenbereichen 14, 15 ist dann mittelbar über den Patienten hergestellt. In diesem Zustand ist ein Stromfluss zwischen den beiden Elektrodenbereichen 14, 15 mittelbar über den Patienten möglich.

Das elektrochirurgische System ist dazu eingerichtet, mittels des elektrochirurgischen Instruments 12 biologisches Gewebe 16 eines Patienten zu behandeln. Bei dem biologischen Gewebe 16 kann es sich um Organgewebe, Muskelgewebe, Fettgewebe, Hautgewebe, Gefäße oder anderes biologisches Gewebe 16 des Patienten handeln. Beispielsgemäß kann mittels des elektrochirurgischen Instruments 12 das Gewebe 16 koaguliert und/oder geschnitten werden.

Zum Anschluss des elektrochirurgischen Instruments 12 (monopolares Instrument 12a oder bipolares Instrument 12b) sowie zum Anschluss der Neutralelektrode 13 weist das Versorgungsgerät 11 beim Ausführungsbeispiel mehrere elektrische Geräteanschlüsse 20 auf. Die Anzahl der Geräteanschlüsse 20 kann variieren. Das Versorgungsgerät 11 hat zumindest einen Arbeitsanschluss zum Anschließen des elektrochirurgischen Instruments 12. Bei dem hier veranschaulichten Ausführungsbeispiel weist das Versorgungsgerät 11 zumindest zwei Arbeitsanschlüsse auf, nämlich einen ersten Arbeitsanschluss 21 sowie einen zweiten Arbeitsanschluss 22, sodass sowohl monopolare Instrumente 12a, als auch bipolare Instrumente 12b angeschlossen werden können. An den ersten Arbeitsanschluss 21 kann die Arbeitselektrode 23 des monopolaren Instruments 12a angeschlossen werden. Bei der Verwendung eines bipolaren Instruments 12b kann eine erste Arbeitselektrode 24 an den ersten Arbeitsanschluss 21 und eine zweite Arbeitselektrode 25 an den zweiten Arbeitsanschluss 22 angeschlossen werden.

Außerdem gehören zu den Geräteanschlüssen 20 des Versorgungsgeräts 11 beim Ausführungsbeispiel ein erster Neutralanschluss 26 zur elektrischen Verbindung mit dem ersten Elektrodenbereich 14 der Neutralelektrode 13 sowie ein zweiter Neutralanschluss 27 zur elektrischen Verbindung mit dem zweiten Elektrodenbereich 15 der Neutralelektrode 13.

Wenn das Versorgungsgerät 11 ausschließlich zur Verwendung mit monopolaren Instrumenten 12b eingerichtet ist, ist in Abwandlung zum veranschaulichten Ausführungsbeispiel ein einziger Arbeitsanschluss 21 ausreichend. Es ist außerdem möglich, mehr als zwei Arbeitsanschlüsse 21, 22 vorzusehen, wenn das elektrochirurgische Instrument 12 mehr als zwei Arbeitselektroden aufweist.

Das Versorgungsgerät 11 ist dazu eingerichtet, die wenigstens eine Arbeitselektrode 23 bzw. 24, 25 des angeschlossenen elektrochirurgischen Instruments 12 mit elektrischer Energie zu versorgen. Während der Behandlung des Gewebes 16 fließt hierbei ein Behandlungsstrom durch das behandelte biologische Gewebe 16. Der Behandlungsstrom kann bei der Anwendung eines monopolaren Instruments 12a von der Arbeitselektrode 23 durch das Gewebe 16 zur Neutralelektrode 13 und von dort zurück zum Versorgungsgerät 11 fließen. Bei der Verwendung eines bipolaren Instruments 12b fließt der Behandlungsstrom zwischen den beiden Arbeitselektroden 24, 25 durch das Gewebe 16. In beiden Fällen wird ein geschlossener Stromkreis hergestellt.

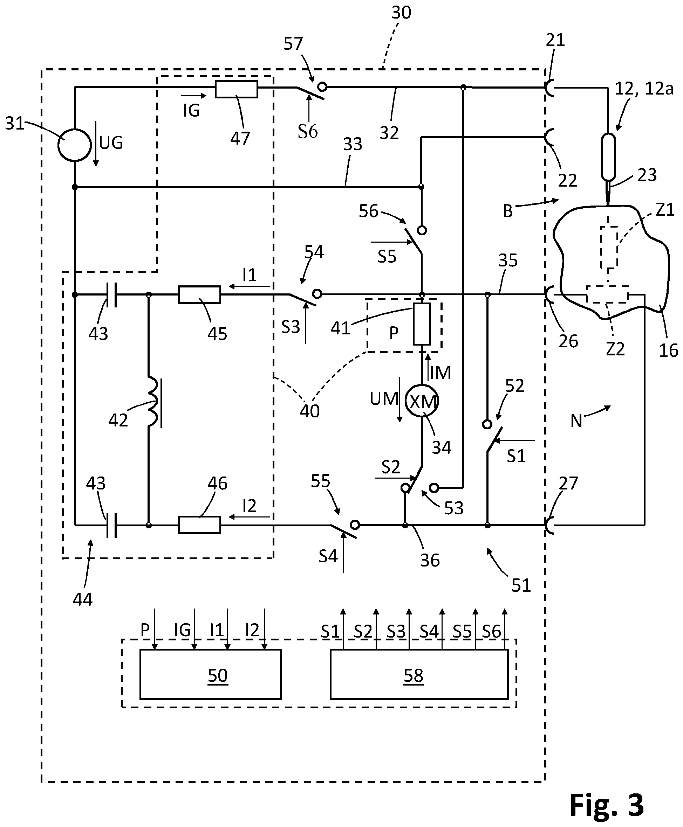

In Figur 3 ist ein Blockschaltbild eines Ausführungsbeispiels einer Betriebsschaltung 30 des Versorgungsgeräts 11 veranschaulicht. Die Betriebsschaltung 30 gemäß Figur 3 stellt die wesentlichen Komponenten und Bestandteile der elektrischen Schaltung des Versorgungsgeräts 11 dar, wobei zusätzliche weitere Schaltungsteile vorhanden sein können, die in der Betriebsschaltung 30 gemäß Figur 3 nicht dargestellt sind.

Die Betriebsschaltung 30 weist einen Generator 31 auf, der als Wechselstromquelle oder Wechselspannungsquelle ausgebildet sein kann. Über den Generator 31 wird beispielsgemäß ein Wechselstrom und/oder eine Wechselspannung mit alternierender Polarität als Generatorspannung UG und/oder Generatorstroms IG bereitgestellt, wobei die Frequenz im Bereich von 300 kHz bis 4,0 MHz liegen kann. Abhängig vom Betriebsmodus des Versorgungsgeräts 11 bzw. des elektrochirurgischen Systems 10 und der Anwendung kann der Generator 31 die Generatorspannung UG bzw. den Generatorstrom IG auch bei unterschiedlichen Frequenzen bereitstellen.

Wie es in Figur 3 dargestellt ist, ist ein Anschluss des Generators 31 über eine erste Leitung 32 mit dem ersten Arbeitsanschluss 21 elektrisch verbunden und der Generator 31 ist mit seinem anderen Anschluss über eine zweite Leitung 33 elektrisch mit dem zweiten Arbeitsanschluss 22 des Versorgungsgeräts 11 verbunden.

Die Betriebsschaltung 30 hat beispielsgemäß eine Messsignalquelle 34 zur Bereitstellung eines Messsignals XM. Die Messsignalquelle 34 kann eine Stromquelle oder eine Spannungsquelle sein und dementsprechend als Messsignal XM eine Messspannung UM und/oder einen Messstrom IM bereitstellen. Bei dem Messsignal XM handelt es sich um ein Wechselstrom- bzw. Wechselspannungssignal mit einer Messfrequenz fm, insbesondere mit alternierender Polarität. Die Messfrequenz f kann auf verschiedene Messfrequenzen fm stufenlos oder in Stufen eingestellt werden, z.B. eine erste Messfrequenz f1, eine zweite Messfrequenz f2 und/oder eine dritte Messfrequenz f3. Die Anzahl der einstellbaren Messfrequenzen fm kann variieren. Beim Ausführungsbeispiel kann die Messfrequenz f auf eine oder mehrere Messfrequenzen fm im Bereich von 1,0 Hz oder 10 Hz oder 100 Hz bis 1,0 MHz eingestellt werden.

Das Messsignal XM (z.B. Messspannung UM) hat eine Amplitude von maximal 1,0 V und beim Ausführungsbeispiel von etwa 10 mV.

Die Messsignalquelle 34 ist mit einem Anschluss über eine dritte Leitung 35 an den ersten Neutralanschluss 26 und mit dem anderen Anschluss über eine vierte Leitung 36 mit dem zweiten Neutralanschluss 27 elektrisch verbunden. Somit kann die Messsignalquelle 34 einen Stromfluss eines Messstromes IM durch eine an die Neutralanschlüsse 26, 27 angeschlossene Neutralelektrode 13 bewirken.

Die Betriebsschaltung 30 weist außerdem eine Messeinrichtung 40 auf. Die Messeinrichtung 40 ist dazu eingerichtet, einen Impedanzparameter P zu messen, der eine Impedanz Z zwischen zwei ausgewählten Geräteanschlüssen 20 beschreibt, zwischen denen die Messspannung UM anliegt. Beim Ausführungsbeispiel kann der Impedanzparameter P eine erste Impedanz Z1 in einem Behandlungsstrompfad B oder eine zweite Impedanz Z2 in einem Neutralstrompfad N beschreiben. Der Behandlungsstrompfad B ist die elektrische Verbindung zwischen dem ersten Arbeitsanschluss 21 und den Neutralanschlüssen 26, 27 über das monopolare Instrument 12a und das Gewebe 16 oder zwischen dem ersten Arbeitsanschluss 21 und dem zweiten Arbeitsanschluss 22 über das bipolare Instrument 12b und das Gewebe 16. Der Neutralstrompfad N ist die elektrische Verbindung zwischen den Neutralanschlüssen 26, 27 über die Neutralelektrode 13 und den Patienten.

Der Generatorstrom oder dessen Anteil, der durch einen das Instrument aufweisenden Behandlungsstrompfad B fließt, kann auch als Arbeits- oder Behandlungsstrom bezeichnet werden.

Wenn als Messsignal XM eine Messspannung UM bereitgestellt wird, kann ein dadurch verursachter Messstrom IM durch den Behandlungsstrompfad B oder den Neutralstrompfad N als Impedanzparameter P verwendet werden. Wird umgekehrt als Messsignal XM ein Messstrom IM eingeprägt, kann als Impedanzparameter P eine dadurch am Behandlungsstrompfad B oder am Neutralstrompfad N anliegende Messspannung UM als Impedanzparameter P verwendet werden. Bei dem in Figur 3 dargestellten Beispiel dient als Impedanzparameter P der Messstrom IM, der beispielsweise anhand der Spannung an einem in Reihe zur Messsignalquelle 34 geschalteten ersten Messwiderstand 41 der Messeinrichtung 40 gemessen werden kann.

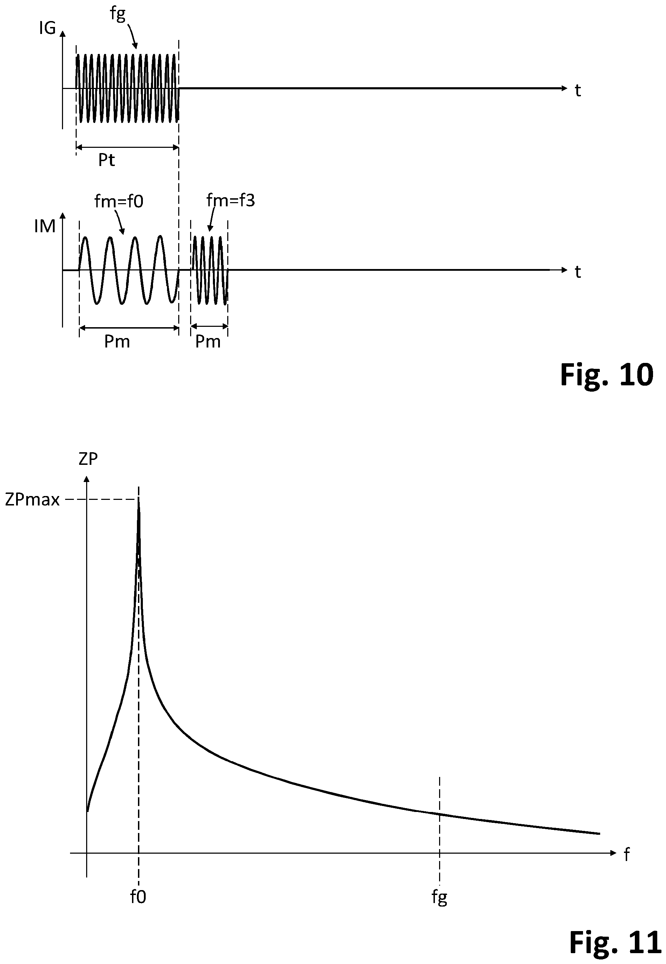

Beim Ausführungsbeispiel hat die Messeinrichtung 40 außerdem eine frequenzabhängige Impedanzschaltung, die hier als Parallelschwingkreis 44 ausgeführt ist. der Die frequenzabhängige Impedanzschaltung und beispielsgemäß der Parallelschwingkreis 44 verbindet die dritte Leitung 35 mit der vierten Leitung 36 elektrisch. In einer bevorzugten Ausführung kann der Parallelschwingkreis 44 eine Parallelinduktivität 42 zwei Serienkondensatoren 43aufweisen. Weitere Bauteile sind optional, aber nicht erforderlich. Die Werte der Bauteile des Parallelschwingkreises 44 können dabei so ausgelegt werden, dass der Schwingkreis einen frequenzabhängigen Impedanzverlauf aufweist, wie er beispielhaft in Figur 11 gezeigt ist. Der Impedanzverlauf ist der Betrag der Impedanz ZP des Parallelschwingkreises 44 abhängig von der Frequenz f dargestellt. Der Impedanzverlauf hat ein Impedanzmaximum ZPmax (Absolutwert) bei einer zur Unterscheidbarkeit als Schwingkreisfrequenz f0 bezeichneten Frequenz, die signifikant verschieden ist von der Generatorfrequenz fg, mit der der Generator 31 die Generatorspannung UG bzw. den Generatorstrom IG (kann auch als Arbeitsstrom bezeichnet werden) bereitstellt. In einer bevorzugten Ausführungsform ist die Schwingkreisfrequenz f0 um mindestens einen Faktor drei kleiner als die Generatorfrequenz fg. Die vom Parallelschwingkreis 44 gebildete frequenzabhängige Impedanzschaltung ist darüber hinaus dadurch gekennzeichnet, dass sie bei der Generatorfrequenz fg einen gegenüber dem Impedanzmaximum ZPmax deutlich niedrigeren Betrag bzw. Absolutwert der Impedanz ZP aufweist, beispielsweise maximal 10% vom Impedanzmaximum ZPmax.

Anstelle des Parallelschwingkreises können auch andere frequenzabhängige Impedanzschaltungen mit einem oder mehreren lokalen Impedanzmaximum verwendet werden, die wenigstens ein Bauteil mit einem frequenzanhängigen Impedanzbetrag aufweisen, insbesondere wenigstens eine Spule bzw. Impedanz und/oder wenigstens einen Kondensator.

Die Messeinrichtung 40 kann zusätzlich zum Impedanzparameter P weitere Parameter messen, beispielsweise einen ersten Strom I1 durch die dritte Leitung 35 und/oder einen zweiten Strom I2 durch die vierte Leitung 36. Hierzu kann die Messeinrichtung 40 einen zweiten Messwiderstand 45 in der dritten Leitung 35 und einen dritten Messwiderstand 46 in der vierten Leitung 36 aufweisen. Weiter optional kann die Messeinrichtung 40 auch zur Messung eines Generatorstromes IG sein, der aufgrund einer vom Generator 31 bereitgestellten Generatorspannung UG fließt. Beispielsweise kann hierzu ein vierter Messwiderstand 47 in der ersten Leitung 32 angeordnet sein.

Zu der Betriebsschaltung 30 gehört beim Ausführungsbeispiel außerdem eine Auswerteeinheit 50. Der Auswerteeinheit 50 wird zumindest der Impedanzparameter P zur Verfügung gestellt, der durch die Messeinrichtung 40 gemessen wird. Der Impedanzparameter P beschreibt, wie erläutert, die ersten Impedanz Z1 im Behandlungsstrompfad B und/oder die zweite Impedanz Z2 im Neutralstrompfad N.

Optional können der Auswerteeinheit 50 auch weitere Parameter oder Variablen bereitgestellt werden, die durch die Messeinrichtung 40 erfasst werden, wie z.B. der erste Strom I1 und/oder der zweite Strom I2 und/oder der Generatorstrom IG.

Die Betriebsschaltung 30 weist außerdem eine zwischen mehreren Schaltzuständen umschaltbare Schalteinrichtung 51 auf. Die Schalteinrichtung 51 kann dazu einen oder mehrere ansteuerbare Schalter aufweisen, die jeweils über ein zugeordnetes Steuersignal ansteuerbar sind. Beim Ausführungsbeispiel sind ein durch ein erstes Steuersignal S1 ansteuerbarer erster Schalter 52, ein durch ein zweites Steuersignal S2 ansteuerbarer zweiter Schalter 53, ein durch ein drittes Steuersignal S3 ansteuerbarer dritter Schalter 54, ein durch ein viertes Steuersignal S4 ansteuerbarer vierter Schalter 55, ein durch ein fünftes Steuersignal S5 ansteuerbarer fünfter Schalter 56, sowie ein durch ein sechstes Steuersignal S6 ansteuerbarer sechster Schalter 57 vorhanden. Die Steuersignale S1-S6 werden von einer Steuereinheit 58 der Betriebsschaltung 30 erzeugt. Wie es schematisch in Figur 3 dargestellt ist, können die Auswerteeinheit 50 und die Steuereinheit 58 optional eine gemeinsame Komponente oder Baugruppe sein.

Die Schalter 52-57 können beispielsweise als steuerbare Halbleiterschalter (z.B. Bipolar- oder Feldeffekttransistoren) ausgebildet sein. Es versteht sich, dass die Schalteinrichtung 51 in Abwandlung zum dargestellten Ausführungsbeispiel gemäß Figur 3 auch mehr oder weniger als sechs steuerbare Schalter 52 bis 57 aufweisen kann. Bei dem Ausführungsbeispiel sind die Schalter 52 bis 57 der Schalteinrichtung 51 wie folgt angeordnet:

Der erste Schalter 52 ist in einem Verbindungspfad zwischen der dritten Leitung 35 bzw. dem ersten Neutralanschluss 26 und der vierten Leitung 36 bzw. dem zweiten Neutralanschluss 27 angeordnet und kann die beiden Neutralanschlüsse 26, 27 niederohmig miteinander verbinden bzw. kurzschließen.

Der zweite Schalter 53 ist mit einer Anschlussseite der Messsignalquelle 34 verbunden und kann diese Anschlussseite der Messsignalquelle in einer Schaltstellung mit der vierten Leitung 36 bzw. dem zweiten Neutralanschluss 27 elektrisch verbinden, wo sie in einer anderen Schaltstellung mit der ersten Leitung 32 bzw. dem ersten Arbeitsanschluss 21 elektrisch verbinden. Die dem zweiten Schalter 53 entgegengesetzte Anschlussseite der Messsignalquelle 34 ist mit der dritten Leitung 35 bzw. dem ersten Neutralanschluss 26 elektrisch verbunden.

Der dritte Schalter 54 ist zwischen dem ersten Neutralanschluss 26 und der einen Anschlussseite des Parallelschwingkreises 44 in der ersten Leitung 35 angeordnet. Der vierte Schalter 55 ist zwischen dem zweiten Neutralanschluss 27 und der anderen Seite des Parallelschwingkreises 44 in der zweiten Leitung 36 angeordnet. Der dritte Schalter 54 und der vierte Schalter 55 können die elektrische Verbindung zwischen dem Parallelschwingkreises 44 und dem jeweils zugeordneten Neutralanschluss 26 bzw. 27 respektive der Messsignalquelle 34 herstellen oder unterbrechen.

Der fünfte Schalter 56 ist auf der dem zweiten Schalter 53 entgegengesetzten Seite der Messsignalquelle 34 in Reihe zur Messsignalquelle 34 geschaltet und kann eine elektrische Verbindung zwischen der Messsignalquelle 34 und der zweiten Leitung 33 bzw. dem zweiten Arbeitsanschluss 22 herstellen oder unterbrechen.

Der sechste Schalter 57 ist zwischen dem vierten Messwiderstand 47 und dem ersten Arbeitsanschluss 21 angeordnet und kann eine elektrische Verbindung vom Generator 31 zum ersten Arbeitsanschluss 21 und zum zweiten Schalter 53 herstellen oder trennen.

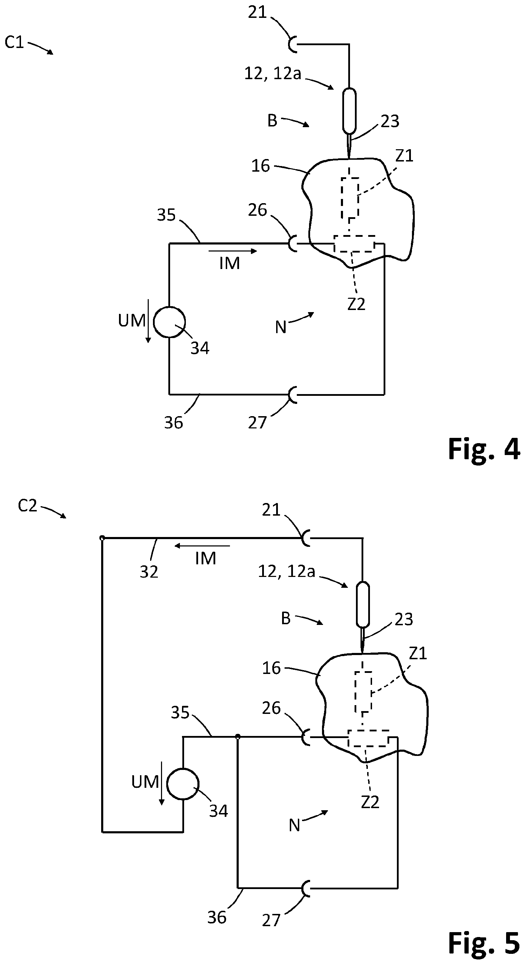

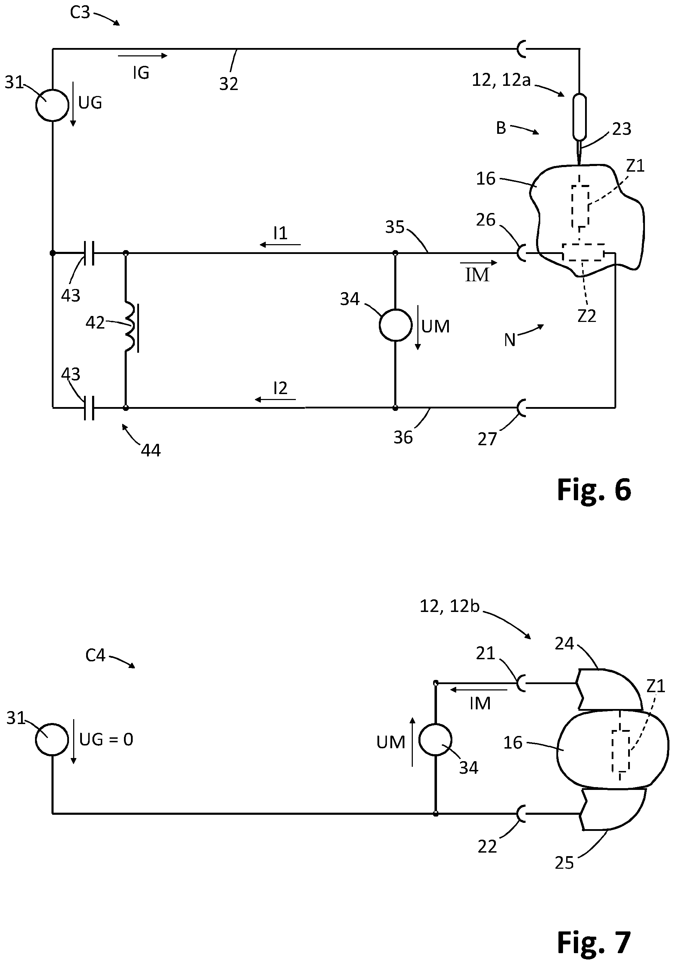

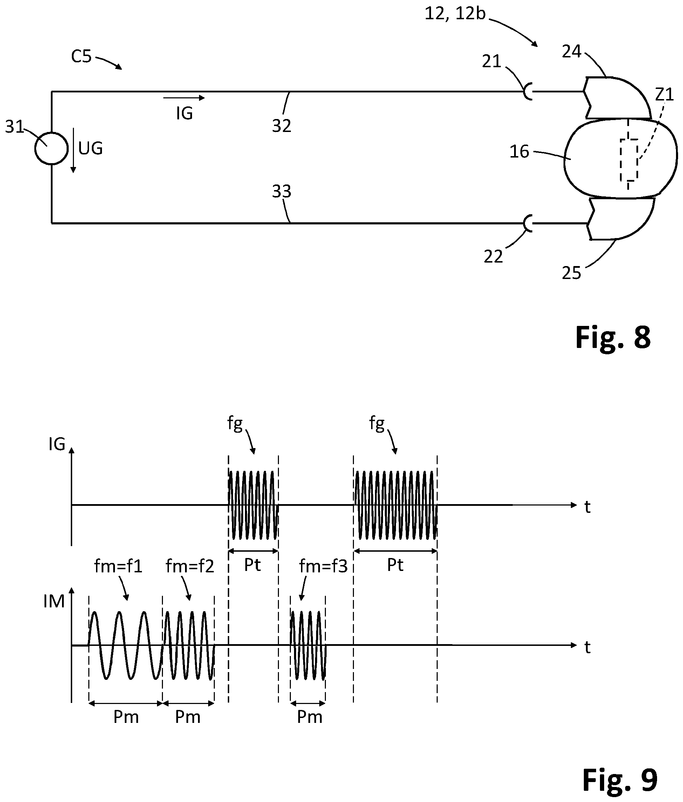

Die Steuereinheit 58 kann die Schalteinrichtung 51 zwischen wenigstens zwei verschiedenen Schaltzuständen umschalten, beispielsgemäß zwischen einem ersten Schaltzustand C1 (Figur 4), einem zweiten Schaltzustand C2 (Figur 5), einem dritten Schaltzustand C3 (Figur 6), einem vierten Schaltzustand C4 (Figur 7) und einem fünften Schaltzustand C5 (Figur 8). Die Ansteuerung der Schalteinrichtung 51 kann unter anderem vom aktuellen Betriebszustand des Versorgungsgeräts 11 abhängen, beispielsweise ob das Versorgungsgerät 11 mit einem monopolaren oder bipolaren Instrument 12 zusammenarbeitet und/oder ob zur Behandlung des Gewebes 16 eine elektrische Leistung an der wenigstens einen Arbeitselektrode 23, 24, 25 bereitgestellt wird oder ob eine Behandlungspause ohne Bereitstellung einer solchen elektrischen Leistung an der wenigstens einen Arbeitselektrode 23, 24, 25 vorliegt.

Mittels der unterschiedlichen Schaltzustände C1-C5 kann die Auswerteeinheit 50 verschiedene Auswerteaufgaben ausführen.

Nachfolgend werden die Schaltzustände C1-C5, die die Schalteinrichtung 51 beim Ausführungsbeispiel annehmen kann, anhand der Figuren 4-7 erläutert, wobei in den Figuren 4-7 jeweils nur die wesentlichen Bestandteile der Betriebsschaltung 30 in vereinfachter Weise veranschaulicht sind.

Der erste Schaltzustand C1 ist für die Verwendung mit einem monopolaren Instrument 12a und einer Neutralelektrode 13 vorgesehen. Im ersten Schaltzustand C1 sind der erste Schalter 52, der dritte Schalter 54, der vierte Schalter 55 und der fünfte Schalter 56 in ihrem nicht leitenden Zustand. Der zweite Schalter 53 verbindet die Messsignalquelle 34 mit dem zweiten Neutralanschluss 27. Der Neutralstrompfad N ist der Strompfad zwischen dem ersten Neutralanschluss 26 und dem zweiten Neutralanschluss 27, der durch die Neutralelektrode 13 und das Gewebe 16 gebildet ist und im Ersatzschaltbild anhand der zweiten Impedanz Z2 dargestellt ist. Der Neutralstrompfad N ist über die dritte Leitung 35 und die vierte Leitung 36 mit der Messsignalquelle 34 zu einem geschlossenen Stromkreis verbunden.

Wenn die Messsignalquelle 34 eine Messspannung UM bereitstellt, fließt durch den Neutralstrompfad N ein Messstrom IM, der mittels der Messeinrichtung 51 gemessen und als Impedanzparameter P an die Auswerteeinheit 50 übermittelt werden kann. In der Auswerteeinheit 50 kann geprüft bzw. überwacht werden, ob die zweite Impedanz Z2 einem Prüfkriterium entspricht, das eine ausreichend gute Leitfähigkeit zwischen der Neutralelektrode 13 und dem Patienten beschreibt. Auf diese Weise kann eine für die Behandlung des Patienten erforderliche elektrische Leitfähigkeit zwischen der Neutralelektrode 13 und dem Patienten überwacht werden. Eine nicht korrekt angebrachte Neutralelektrode 13 und/oder ein Ablösen der Neutralelektrode 13 während der Behandlung können erkannt werden. Dadurch lassen sich zu hohe Stromdichten innerhalb des Gewebes im Anschluss an die Neutralelektrode 13 vermeiden, die zu Gewebeschädigungen führen könnten. Erfüllt die anhand des Impedanzparameter P bzw. des Messstromes IM ermittelte zweite Impedanz Z2 des Neutralstrompfades N nicht das vorgegebene Prüfkriterium, kann eine entsprechende Meldung an den Anwender des elektrochirurgischen Systems 10 ausgegeben werden und/oder der Weiterbetrieb und beispielsweise das Versorgen der wenigstens einen Arbeitselektrode 23 bzw. 24, 25 mit elektrischer Energie kann unterbunden werden.

In Figur 5 ist der zweite Schaltzustand C2 veranschaulicht. Der zweite Schaltzustand C2 findet ebenfalls bei einem elektrochirurgischen System 10 Anwendung, das ein monopolares Instrument 12a und eine Neutralelektrode 13 aufweist. Im zweiten Schaltzustand C2 ist der erste Schalter 52 in seinem leitenden Zustand und verbindet sie beiden Neutralanschlüsse 26, 27 niederohmig. Der zweite Schalter 53 ist in einem Schaltzustand, in dem er die Messsignalquelle 34 einerseits mit den Neutralanschlüsse 26, 27 elektrisch verbindet und andererseits mit dem ersten Arbeitsanschluss 21 elektrisch verbindet. Über das monopolare Instrument 12a und dessen Arbeitselektrode 23, das Gewebe 16 sowie die an die Neutralanschlüsse 26, 27 angeschlossene Neutralelektrode 13 (Behandlungsstrompfad B bei Anwendung des monopolaren Instruments 12a) wird der Stromkreis geschlossen.

Im zweiten Schaltzustand C2 kann die Messsignalquelle 34 eine Messspannung UM als Messsignal XM bereitstellen, so dass ein Messstrom IM durch den Behandlungsstrompfad B fließt. Der Behandlungsstrompfad B ist durch die erste Impedanz Z1 charakterisiert, die insbesondere vom Zustand und vom Typ des Gewebes 16 abhängt, mit der die Arbeitselektrode 23 des monopolaren Instruments 12a in elektrisch leitendem Kontakt steht. Der Messstrom IM durch den Behandlungsstrompfad B ist charakteristisch für die erste Impedanz Z1 und kann als Impedanzparameter P in der Auswerteeinheit 50 ausgewertet werden. Die Auswerteeinheit 50 kann anhand des als Impedanzparameter P verwendeten Messstromes IM auf die erste Impedanz Z1 schließen und daraus einen Zustand und/oder einen Typ des behandelten Gewebes 16 bestimmen.

Während einer Messung bei aktivierter Messsignalquelle 34 im ersten Schaltzustand C1 und/oder im zweiten Schaltzustand C2 wird der Generator 31 abgeschaltet, sowie der sechste Schalter 57 geöffnet, so dass weder eine Generatorspannung UG, noch ein Generatorstrom IG bereitgestellt werden und der Generator 31 galvanisch nicht mit der Messquelle XM verbunden ist. Dadurch wird eine Beeinträchtigung der Messung vermieden.

In Figur 6 ist schematisch der dritte Schaltzustand C3 dargestellt. Im dritten Schaltzustand C3 sind der erste Schalter 52 und der fünfte Schalter 56 in nicht leitendem Zustand. Der dritte Schalter 54 und der vierte Schalter 55 sind jeweils in ihrem leitenden Zustand. Der zweite Schalter 53 verbindet die Messsignalquelle 34 mit dem zweiten Neutralanschluss 27.

Im dritten Schaltzustand C3 wird mittels eines monopolaren Instruments 12a Gewebe 16 eines Patienten behandelt. Der Generator 31 ist aktiv und stellt eine Generatorspannung UG und/oder einen Generatorstrom IG bereit, so dass an der Arbeitselektrode 23 des monopolaren Instruments 12a elektrischer Leistung für die Behandlung des Gewebes 16 zur Verfügung steht. Durch den Behandlungsstrompfad B zwischen dem ersten Arbeitsanschluss 21 und den Neutralanschlüssen 26, 27 fließt während der Behandlung ein Strom, der sich in einen ersten Strom I1 in der dritten Leitung 35 und einen zweiten Strom I2 in der vierten Leitung 36 aufteilt. Mittels der Messeinrichtung 51 können die beiden Ströme I1, I2 miteinander verglichen werden, um Ungleichheiten oder Asymmetrien zu erkennen, die beispielsweise aufgrund einer nicht korrekt am Patienten angeordneten Neutralelektrode 13 verursacht werden können. Hierzu können ein oder mehrere charakteristische Stromparameter der Ströme I1, I2 miteinander verglichen werden, wie beispielsweise eine Amplitude und/oder ein Betrag und/oder eine Phasenlage.

Im dritten Schaltzustand C3 wird darüber hinaus während einer Applikation des Gewebes 16 mit Generatorstrom IG mittels der Messsignalquelle 34 ein Messsignal XM (z.B. Messspannung UM) bereitgestellt, wodurch durch den Neutralstrompfad N ein Messstrom IM fließt, der mittels der Messeinrichtung 51 gemessen und als Impedanzparameter P an die Auswerteeinheit 50 übermittelt werden kann. Das Messsignal XM bzw. die Messspannung UM wird dabei bei einer Messfrequenz fm bereitgestellt, die sich ausreichend von der Generatorfrequenz fg des Generatorstroms IG unterscheidet. Vorzugsweise ist die Messfrequenz fm des Messsignals XM bzw. der Messspannung UM in etwa gleich groß wie bei der Schwingkreisfrequenz f0, bei der Parallelschwingkreis 44 sein Impedanzmaximum ZPmax aufweist. Für den Messstrom IM stellt der Parallelschwingkreis 44 einen Widerstand mit hoher Impedanz parallel zur zweiten Impedanz Z2 der Neutralelektrode 13 dar, wodurch die Imedanz der Neutralelektrode basierend auf dem gemessenen Impedanzparameter P sehr genau ermittelt werden kann. Für den Generatorstrom IG stellt der Parallelschwingkreis 44 eine niederohmige Verbindung zwischen den beiden Neutralanschlüssen 26 und 27 her und somit eine elektrische Verbindung über die dritte Leitung 35 und die vierte Leitung 36 zum Generator 31.

Im Unterschied zu den bisher beschriebenen Schaltzuständen C1-C3 betrifft der vierte Schaltzustand C4 (Figur 7) ein elektrochirurgisches System 10 mit einem bipolaren Instrument 12b. Im vierten Schaltzustand C4 sind der erste Schalter 52, der dritte Schalter 54, der vierte Schalter 55 sowie der sechste Schalter 57 in ihrem nicht leitenden Zustand. Der fünfte Schalter 56 ist hingegen in seinem leitenden Zustand. Der zweite Schalter 53 verbindet die Messsignalquelle 34 mit dem ersten Arbeitsanschluss 21 bzw. der ersten Leitung 32. Die Messsignalquelle 34 ist daher zwischen die beiden Arbeitsanschlüsse 21, 22 geschaltet, der Generator 31 ist durch den sechsten Schalter 57 von der Messung getrennt. An den Behandlungspfad B kann somit eine Messspannung UM angelegt werden, so dass ein Messstrom fließt, der als Impedanzparameter P in der Auswerteeinheit 50 ausgewertet werden kann. Der Impedanzparameter P beschreibt in diesem Fall die erste Impedanz Z1 des Behandlungspfades B, die im Wesentlichen durch das Gewebe 16 charakterisiert ist, mit der die erste Arbeitselektrode 24 und die zweite Arbeitselektrode 25 des bipolaren Instruments 12b in elektrisch leitendem Kontakt stehen. Basierend auf dem Impedanzparameter P bzw. der dadurch charakterisierten ersten Impedanz Z1 kann der Zustand und/oder der Typ des Gewebes 16 in der Auswerteeinheit 50 ermittelt werden.

Analog zum Schaltzustand C4 betrifft der fünfte Schaltzustand C5 (Figur 8) ebenfalls ein elektrochirurgisches System 10 mit einem bipolaren Instrument 12b. In diesem fünften Schaltzustand C5 ist der fünfte Schalter 56 in seinem nicht leitenden Zustand und trennt somit die Messsignalquelle 34 vom zweiten Arbeitsanschluss 22. Der sechste Schalter 57 befindet sich im fünften Schaltzustand C5 in seinem leitenden Zustand und schließt dadurch dem Stromkreis vom Generator 31 über den Behandlungsstrompfad B (durch den ersten Arbeitsanschluss 21 und ein bipolares Instrument 12b, das Gewebe 16 und den zweiten Arbeitsanschluss 22). Dieser fünfte Schaltzustand C5 ermöglicht die Behandlung von Gewebe 16 im bipolaren Betrieb.

Im fünften Schaltzustand C5 kann die Messsignalquelle dazu eingerichtet oder derart gesteuert sein, dass sie kein Messsignal XM erzeugt.

In allen Schaltzuständen C1-C5 kann das Ergebnis der Auswertung verwendet werden, um den Betrieb des Versorgungsgeräts 11 automatisch zu beeinflussen und/oder um einem Anwender mittels einer geeigneten Bedienschnittstelle eine Information bereitzustellen, die beispielsweise optisch und/oder akustisch ausgegeben werden kann. Beispielsweise kann der Anwender über den Zustand und/oder den Typ des behandelten Gewebes 16 informiert werden. Abhängig vom Zustand und/oder Typ des Gewebes können auch die elektrische Leistung und/oder die Frequenz des Generators 31 und/oder andere Einstellungen des elektrochirurgischen Systems 10 und insbesondere des Versorgungsgeräts 11 eingestellt oder verändert werden. Hat das elektrochirurgische System 10 ein monopolares Instrument 12a mit einer Neutralelektrode 13, kann zusätzlich die korrekte Anlage der Neutralelektrode 13 am Patienten geprüft und/oder während der Behandlung überwacht werden.

Vorzugsweise wird beim Betrieb des elektrochirurgischen Systems 10 in einem oder mehreren Schaltzuständen, insbesondere dem ersten Schaltzustand C1 und/oder zweiten Schaltzustand C2 und/oder vierten Schaltzustand C4 und/oder fünften Schaltzustand C5, nicht gleichzeitig über den Generator 31 und über die Messsignalquelle 34 ein Wechselstrom bzw. eine Wechselspannung erzeugt. Vielmehr erfolgt die Erzeugung eines Messsignals XM und die Messung des die Impedanz Z1, Z2 charakterisierenden Impedanzparameters P in zeitlichen Phasen, in denen der Generator 31 inaktiv ist und somit keine elektrische Leistung (Generatorspannung UG und/oder Generatorstrom IG) bereitstellt, wie es schematisch in Figur 9 dargestellt ist. Wie es Figur 9 veranschaulicht, wird der Stromfluss eines Messstromes IM nur dann durch Aktivieren der Messsignalquelle 34 verursacht, wenn keine Behandlung des Gewebes 16 durch einen vom Generator 31 verursachten Generatorstrom IG erfolgt. Mit anderen Worten überlappen sich Behandlungsphasen Pt, während denen ein Generatorstrom IG bzw. Behandlungsstrom durch den Behandlungsstrompfad B fließt, in dem einen oder den mehreren Schaltzuständen, insbesondere dem ersten Schaltzustand C1 und/oder zweiten Schaltzustand C2 und/oder vierten Schaltzustand C4 und/oder fünften Schaltzustand C5, nicht mit Messphasen Pm, während denen ein Messstrom IM durch den Neutralstrompfad N oder den Behandlungsstrompfad B fließt.

In Figur 9 ist auch zu erkennen, dass die Messfrequenz fm, mit der das Messsignal XM erzeugt wird, variieren kann. Lediglich beispielhaft sind in Figur 9 drei unterschiedliche Messfrequenzen fm = f1, f2, f3 während verschiedener Messphasen Pm veranschaulicht. Die Anzahl der unterschiedlichen Messfrequenzen fm kann variieren.

In Figur 9 ist ferner zu erkennen, dass sich Messphasen Pm mit unterschiedlichen Messfrequenzen fm = f1, f2, f3 unmittelbar aneinander anschließen können oder durch eine Behandlungsphase Pt voneinander getrennt sein können.

Im dritten Schaltzustand C3 ist im Unterschied zu anderen Schaltzuständen, beispielsgemäß dem ersten Schaltzustand C1 und/oder zweiten Schaltzustand C2 und/oder vierten Schaltzustand C4 und/oder fünften Schaltzustand C5, eine gleichzeitige Aktivierung der Messsignalquelle 34 und des Generators 31 möglich. Dazu wird das Messsignal XM bei einer Messfrequenz fm erzeugt, die sich deutlich von der Generatorfrequenz fg unterscheidet und vorzugsweise in etwa gleich groß ist wie die Schwingkreisfrequenz f0. Wie erläutert stellt der Parallelschwingkreis 44 für den Messstrom IM einen Parallelwiderstand mit großem Betrag der Impedanz ZP im Bereich des Impedanzmaximums ZPmax dar während er für den Arbeitsstrom IG bei der Generatorfrequenz fg eine niederohmige Verbindung darstellt. Dies ermöglicht die Auswertung des Impedanzparameters P bei der Messfrequenz fm = f0 auch während einer Behandlung des Gewebes 16 mit einem monopolaren Instrument 12a. Somit kann beispielsweise eine Ablösung der Neutralelektrode 13 auch während einer Behandlung bzw. Aktivierung des Generators 31 und des monopolaren Instruments 12a überwacht werden.

In Figur 10 ist beispielhaft dargestellt, dass sich eine Behandlungsphase Pt und eine Messphase Pm zeitlich überlappen können, als dass eine gleichzeitige Aktivierung des Generators 31 und der Messsignalquelle 34 erfolgt. Es ist zu erkennen, dass sich die Messfrequenz fm = f0, mit der das Messsignal XM erzeugt wird, erheblich von der Generatorfrequenz fg unterscheidet. Nach einer Aktivierung des Generators 31 kann ein Messsignal XM wieder bei einer anderen Frequenz, wie zur Veranschaulichung in Figur 10 mit f3 illustriert, gemessen werden, die beispielsweise auch ähnlich oder gleich der Generatorfrequenz fg ist, in dem die Schalteinrichtung 51 beispielsweise den Schaltzustand C1 oder C2 einnimmt.

In einer Ausführungsform kann die Messfrequenz fm während einer Aktivierung des Generators 31 in einem kleinen Frequenzband um die Schwingkreisfrequenz f0 des Impedanzmaximums ZPmax der Impedanz ZP des Parallelschwingkreises 44 variiert werden, um die Frequenz, bei der das Impedanzmaximum ZPmax auftritt, exakt zu bestimmen.

In Figur 11 ist ein beispielhafter Impedanzverlauf der Impedanz ZP eines Parallelschwingkreises 44 über einen Frequenzbereich zu sehen. Zu erkennen ist, dass der Parallelschwingkreis 44 bei der Schwingkreisfrequenz f0 hochohmig ist und dort sein Impedanzmaximum ZPmax aufweist. Die Impedanz ZP verringert sich ausgehend von der Schwingkreisfrequenz f0 mit zunehmender und abnehmender Frequenz f und ist bei der Generatorfrequenz fg niederohmig.

Die Erfindung betrifft ein Versorgungsgerät 11 für ein elektrochirurgisches System 10 sowie ein elektrochirurgisches System 10, aufweisend ein erfindungsgemäßes Versorgungsgerät 11. Das elektrochirurgische System 10 kann außerdem ein monopolares Instrument 12a und eine Neutralelektrode 13 oder ein bipolares Instrument 12b aufweisen. Das Versorgungsgerät 11 hat wenigstens einen Arbeitsanschluss 21 für jeweils eine Arbeitselektrode 23, 24, 25 eines elektrochirurgischen Instruments 12, sowie einen ersten Neutralanschluss 26 und einen zweiten Neutralanschluss 27 für eine Neutralelektrode 13. Das Versorgungsgerät 11 hat einen Generator 31, mittels dem eine elektrische Leistung für das elektrochirurgische Instrument 12 zur Behandlung von biologischem Gewebe 16 bereitgestellt werden kann. Außerdem hat das Versorgungsgerät 11 eine Messsignalquelle 34, die ein Messsignal XM in Form einer Wechselspannung und/oder eines Wechselstromes bereitstellt. Ein Impedanzparameter P charakterisierend eine sich aufgrund des Messsignals 34 ergebende Impedanz Z1, Z2 und kann mittels einer Messeinrichtung 40 des Versorgungsgerätes 11 gemessen und zur Auswertung einer Auswerteeinheit 50 des Versorgungsgerätes 11 bereitgestellt werden. Das Versorgungsgerät 11 hat außerdem eine Schalteinrichtung 51, die zwischen mehreren Schaltzuständen C1-C5 umschaltbar ist. In den unterschiedlichen Schaltzuständen C1-C5 lassen sich unterschiedliche Auswertefunktionen mittels der Auswerteeinheit 50 realisieren: Die Messsignalquelle 34 kann sowohl ein Messsignal an einen Behandlungsstrompfad B an einem Arbeitsanschluss 21 als auch an einen Neutralstrompfad N zwischen den Neutralanschlüssen 26, 27 anlegen. Dadurch kann mittels des Messsignals XM sowohl ein behandeltes Gewebe 16 analysiert als auch ein Kontakt zwischen der Neutralelektrode 13 und dem Patienten geprüft bzw. überwacht werden.

### Bezugszeichenliste:

- 10: elektrochirurgisches System
- 11: Versorgungsgerät
- 12: elektrochirurgisches Instrument
- 12a: monopolares Instrument
- 12b: bipolares Instrument
- 13: Neutralelektrode
- 14: erster Elektrodenbereich
- 15: zweiter Elektrodenbereich
- 16: Gewebe

- 20: Geräteanschlüsse
- 21: erster Arbeitsanschluss
- 22: zweiter Arbeitsanschluss
- 23: Arbeitselektrode des monopolaren Instruments
- 24: erste Arbeitselektrode des bipolaren Instruments
- 25: zweite Arbeitselektrode des bipolaren Instruments
- 26: erster Neutralanschluss
- 27: zweiter Neutralanschluss

- 30: Betriebsschaltung
- 31: Generator
- 32: erste Leitung
- 33: zweite Leitung
- 34: Messsignalquelle
- 35: dritte Leitung
- 36: vierte Leitung

- 40: Messeinrichtung
- 41: erster Messwiderstand
- 42: Parallelinduktivität
- 43: Serienkondensator
- 44: Parallelschwingkreis
- 45: zweiter Messwiderstand
- 46: dritter Messwiderstand
- 47: vierter Messwiderstand

- 50: Auswerteeinheit
- 51: Schalteinrichtung
- 52: erster Schalter
- 53: zweiter Schalter
- 54: dritter Schalter
- 55: vierter Schalter
- 56: fünfter Schalter
- 57: sechster Schalter
- 58: Steuereinheit

- B: Behandlungsstrompfad
- C1: erster Schaltzustand
- C2: zweiter Schaltzustand
- C3: dritter Schaltzustand
- C4: vierter Schaltzustand
- f: Frequenz
- f0: Schwingkreisfrequenz
- f1: erste Messfrequenz
- f2: zweite Messfrequenz
- f3: dritte Messfrequenz
- fg: Generatorfrequenz
- fm: Messsfrequenz
- I1: erster Strom
- I2: zweiter Strom
- IG: Generatorstrom
- IM: Messstrom
- N: Neutralstrompfad
- P: Impedanzparameter
- Pm: Messphase
- Pt: Behandlungsphase
- S1: erstes Steuersignal
- S2: zweites Steuersignal
- S3: drittes Steuersignal
- S4: viertes Steuersignal
- S5: fünftes Steuersignal
- S6: Sechstes Steuersignal
- t: Zeit
- UG: Generatorspannung
- UM: Messspannung
- XM: Messsignal
- Z1: erste Impedanz
- Z2: zweite Impedanz
- ZP: Impedanz des Parallelschwingkreises
- ZPmax: Impedanzmaximum der Impedanz des Parallelschwingkreises

## Patentansprüche

1. Versorgungsgerät (11) für ein elektrochirurgisches System (10) aufweisend:
mehrere Geräteanschlüsse (20) aufweisend zwei Neutralanschlüsse (26, 27) für eine Neutralelektrode (13) und wenigstens einen Arbeitsanschluss (21, 22) für jeweils eine Arbeitselektrode (23, 24, 25) eines elektrochirurgischen Instruments (12),
einen Generator (31) zur Bereitstellung einer Generatorspannung (UG) und/oder eines Generatorstromes (IG) mit einer Generatorfrequenz (fg),
eine Messsignalquelle (34) zur Bereitstellung eines mit einer Messfrequenz (fm) alternierenden Messsignals (XM),
eine Messeinrichtung (40) zur Messung eines Impedanzparameters (P), der eine Impedanz (Z1, Z2) zwischen zwei Geräteanschlüssen (20) beschreibt, denen das Messsignal (XM) bereitgestellt wird,
eine zwischen mehreren Schaltzuständen (C1, C2, C3, C4, C5) umschaltbare Schalteinrichtung (51), die in einem ersten Schaltzustand (C1) die Messsignalquelle (34) elektrisch mit den zwei Neutralanschlüssen (26, 27) verbindet und die Messsignalquelle (34) elektrisch vom zumindest einen Arbeitsanschluss (21, 22) trennt, und die in einem zweiten Schaltzustand (C2) die Messsignalquelle (34) elektrisch mit dem zumindest einen Arbeitsanschluss (21, 22) verbindet, und
eine Auswerteeinheit (50), die mit der Messeinrichtung (40) kommunikationsverbunden zur Auswertung des gemessenen Impedanzparameters (P) eingerichtet ist.

2. Versorgungsgerät nach Anspruch 1, aufweisend einen ersten Arbeitsanschluss (21) zum Anschließen einer Arbeitselektrode (23) eines monopolaren Instruments (12a).

3. Versorgungsgerät nach Anspruch 1 oder 2, aufweisend einen ersten Arbeitsanschluss (21) zum Anschließen einer ersten Arbeitselektrode (24) und einen zweiten Arbeitsanschluss (22) zum Anschließen einer zweiten Arbeitselektrode (25) eines bipolaren Instruments (12b).

4. Versorgungsgerät nach einem der vorhergehenden Ansprüche, wobei die Messsignalquelle (34) dazu eingerichtet ist, das Messsignal (XM) bei mehreren voneinander verschiedenen Messfrequenzen (fm) bereitzustellen.

5. Versorgungsgerät nach einem der vorhergehenden Ansprüche, wobei die Auswerteeinheit (50) dazu eingerichtet ist, im ersten Schaltzustand (C1) basierend auf dem gemessenen Impedanzparameter (P) einen elektrischen Kontakt zwischen einer an die zwei Neutralanschlüssen (26, 27) angeschlossenen Neutralelektrode (12) und einem Patienten zu prüfen.

6. Versorgungsgerät nach einem der vorhergehenden Ansprüche, aufweisend einen ersten Neutralanschluss (26) zum Anschließen eines elektrisch leitfähigen ersten Elektrodenbereichs (14) einer Neutralelektrode (13) und einen zweiten Neutralanschluss (27) zum Anschließen eines elektrisch leitfähigen zweiten Elektrodenbereichs (15) der Neutralelektrode (13).

7. Versorgungsgerät nach einem der vorhergehenden Ansprüche, wobei die Auswerteeinheit (50) dazu eingerichtet ist, im zweiten Schaltzustand (C2) basierend auf dem gemessenen Impedanzparameter (P) einen Gewebezustand und/oder Gewebetyp zu bestimmen, mit dem ein an den zumindest einen Arbeitsanschluss (21, 22) angeschlossene Arbeitselektrode (23, 24, 25) eines elektrochirurgischen Instruments (12) in elektrisch leitendem Kontakt ist.

8. Versorgungsgerät nach einem der vorhergehenden Ansprüche, wobei die Schalteinrichtung (51) im ersten Schaltzustand (C1) und im zweiten Schaltzustand (C2) den Generator (31) elektrisch von der Messsignalquelle (34) trennt.

9. Versorgungsgerät nach einem der vorhergehenden Ansprüche, wobei der Generator (31) dazu eingerichtet ist oder derart gesteuert wird, dass der Generator (31) keine Generatorspannung (UG) und keinen Generatorstrom (IG) bereitstellt, wenn die Messsignalquelle (34) im ersten Schaltzustand und/oder im zweiten Schaltzustand (C2) der Schalteinrichtung (51) ein Messsignal (XM) bereitstellt.

10. Versorgungsgerät nach einem der vorhergehenden Ansprüche, wobei die Schalteinrichtung (51) im zweiten Schaltzustand (C2) die Messsignalquelle (34) elektrisch einerseits mit dem zumindest einen Arbeitsanschluss (21, 22) und andererseits den zwei Neutralanschlüssen (26, 27) verbindet.

11. Versorgungsgerät nach einem der vorhergehenden Ansprüche, wobei die Schalteinrichtung (51) in einem dritten Schaltzustand (C3) den Generator (31) elektrisch einerseits mit dem zumindest einen Arbeitsanschluss (21, 22) und andererseits mit den Neutralanschlüssen (26, 27) verbindet.

12. Versorgungsgerät nach Anspruch 11, wobei die Auswerteeinheit (50) dazu eingerichtet ist, im dritten Schaltzustand (C3) einen durch den Generatorstrom (IG) am ersten Neutralanschluss (26) verursachten ersten Strom (I1) und/oder einen am zweiten Neutralanschluss (27) verursachten zweiten Strom (I2) zu prüfen.

13. Versorgungsgerät nach Anspruch 11 oder 12, wobei die Schalteinrichtung (51) im dritten Schaltzustand (C3) eine frequenzabhängige Impedanzschaltung, insbesondere einen Parallelschwingkreis (44), elektrisch mit der Messsignalquelle (34) verbindet.

14. Versorgungsgerät nach einem der Ansprüche 11 bis 13, wobei im dritten Schaltzustand (C3) die Messsignalquelle (34) dazu eingerichtet ist oder derart gesteuert wird, dass die Messsignalquelle (34) ein Messsignal (XM) bei der Messfrequenz (fm) erzeugt, die sich von der Generatorfrequenz (fg) unterscheidet.

15. Versorgungsgerät nach Anspruch 14, wobei im dritten Schaltzustand (C3) die Messsignalquelle (34) dazu eingerichtet ist oder derart gesteuert wird, dass die Messfrequenz (fm) des Messsignals (XM) in einem Frequenzbereich variiert, um eine Frequenz zu ermitteln, bei der die frequenzabhängige Impedanzschaltung ein Impedanzmaximum (ZP)aufweist.

16. Versorgungsgerät nach einem der vorhergehenden Ansprüche, aufweisend einen ersten Arbeitsanschluss (21) und eine zweiten Arbeitsanschluss (22), wobei die Schalteinrichtung (51) in einem vierten Schaltzustand (C4) die Messsignalquelle (34) elektrisch einerseits mit dem ersten Arbeitsanschluss (21) und andererseits mit dem zweiten Arbeitsanschluss (22) verbindet.

17. Versorgungsgerät nach einem der vorhergehenden Ansprüche, aufweisend einen ersten Arbeitsanschluss (21) und eine zweiten Arbeitsanschluss (22), wobei die Schalteinrichtung (51) in einem fünften Schaltzustand (C5) den Generator (31) elektrisch einerseits mit dem ersten Arbeitsanschluss (21) und andererseits mit dem zweiten Arbeitsanschluss (22) verbindet und die Messsignalquelle (34) vom ersten Arbeitsanschluss (21) und vom zweiten Arbeitsanschluss (22) trennt.

18. Elektrochirurgisches System (10) aufweisend ein Versorgungsgerät (11) nach einem der vorhergehenden Ansprüche und ein bipolares Instrument (12b) oder ein monopolares Instrument (12a).
